(19) Europäisches Patentamt
European Patent Office
Office européen des brevets

(11) Numéro de publication: **0 221 820 B1**

# (12) FASCICULE DE BREVET EUROPEEN

(45) Date de publication du fascicule du brevet: **03.01.90**

(51) Int. Cl.⁴: **C07D 413/06**

(21) Numéro de dépôt: **86402419.5**

(22) Date de dépôt: **29.10.86**

(54) **Dérivés de l'imino-3 pyridazine, procédé d'obtention et compositions pharmaceutiques en contenant.**

(30) Priorité: **30.10.85 FR 8516157**

(43) Date de publication de la demande:
**13.05.87 Bulletin 87/20**

(45) Mention de la délivrance du brevet:
**03.01.90 Bulletin 90/1**

(84) Etats contractants désignés:
**AT BE CH DE ES FR GB GR IT LI LU NL SE**

(56) Documents cités:
**Néant.**

(73) Titulaire: **SANOFI, 40, Avenue George V, F-75008 Paris(FR)**

(72) Inventeur: **Wermuth, Camille George, 3, rue de la Cote d'Azur, F-67100 Strasbourg(FR)**
Inventeur: **Schlewer, Gilbert, 1, rue de l'Ecole, F-67400 Ostwald(FR)**
Inventeur: **Heaulme, Michel, 18, rue Gaston Bonheur, F-34670 Baillargues(FR)**

(74) Mandataire: **Gillard, Marie-Louise et al, Cabinet Beau de Loménie 55, Rue d'Amsterdam, F-75008 Paris(FR)**

**EP 0 221 820 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

Dérivés de l'imino-3 pyridazine, procédé d'obtention et compositions pharmaceutiques en contenant.

La présente invention a pour objet de nouveaux dérivés de la dihydro-2,3 imino-3 pyridazine substitués sur l'azote en position 2par un groupe isoxazolylméthyle ou un groupe isothiazolylméthyle.

Les composés selon l'invention répondent à la formule générale :

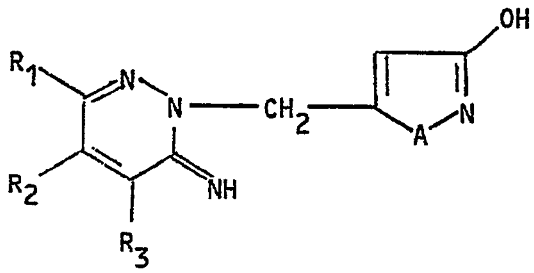

(I)

dans laquelle :

- A représente un atome d'oxygène ou de soufre;
- $R_1$ représente un groupe alkyle inférieur ou un groupe aromatique choisi parmi :
- le groupe phényle;
- les groupes phényle mono- ou poly-substitués par un groupe halogène, un groupe alkyle inférieur, un groupe alcoxy inférieur, un groupe nitro, un groupe hydroxy ou méthylènedioxy;
- le groupe naphtyle;
- le groupe furyle;
- le groupe thiényle ou le groupe pyridyle;
- $R_2$ et $R_3$ désignent chacun indépendamment l'hydrogène ou un groupe alkyle inférieur; un groupe phényle ou encore $R_2$ et $R_3$ pris ensemble constituent, avec les 2 atomes du cycle pyridazinique auxquels ils sont liés, un noyau benzénique.

Les sels que forment les composés de formule (I) avec les acides pharmaceutiquement acceptables font partie intégrante de l'invention. Une étude radiocristallographique effectuée sur les sels des composés de formule (I) montre que la double liaison du groupe imino est entièrement délocalisée. Par suite, les sels peuvent exister sous deux formes tautomères :

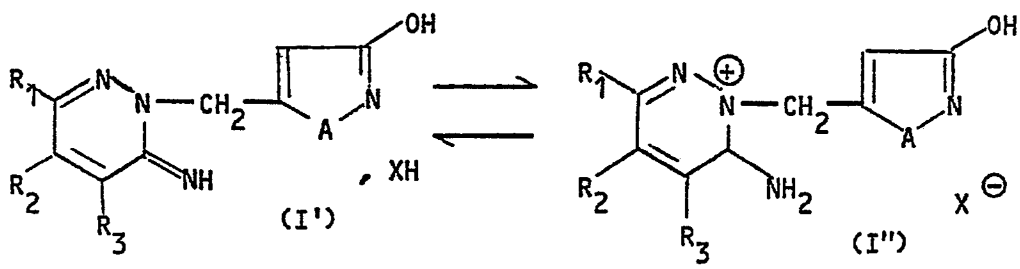

où $R_1$, $R_2$, $R_3$ et A sont tels que définis précédemment et XH représente un acide minéral ou organique, par exemple d'un acide halogénohydrique.

Ces deux formes tautomères font partie de l'invention.

Dans la présente demande, on entend par alkyle inférieur un groupe alkyle ayant de 1 à 4 atomes de carbone. Par groupe alcoxy inférieur, on entend un groupe -0-alkyle inférieur.

Les composés selon l'invention peuvent être préparés par action d'un méthoxy-3 halogénométhyl-5 isoxazole ou isothiazole 2 sur une amino-3 pyridazine convenablement substituée 1 selon le schéma réactionnel :

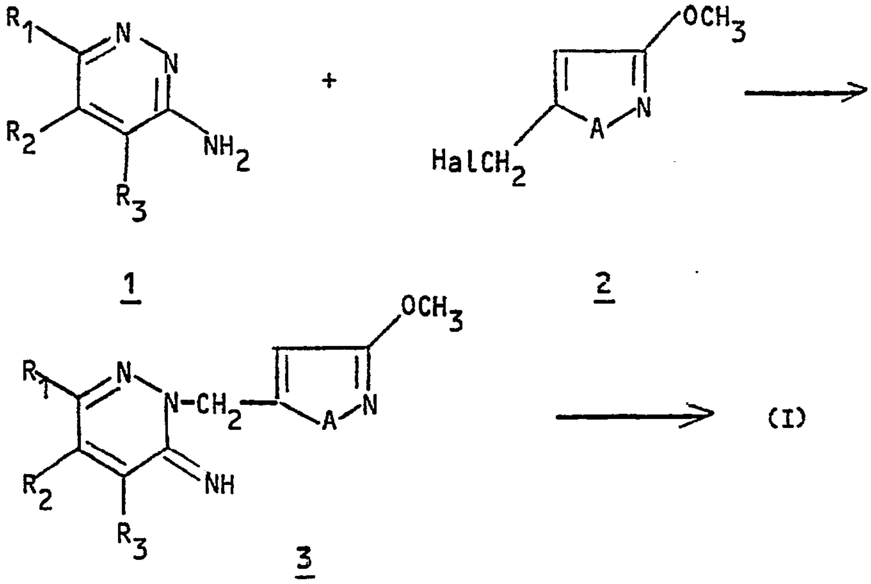

1 2

3

(I)

On obtient ainsi les composés 0-méthylés 3.

La réaction s'effectue par chauffage des deux réactifs au sein d'un solvant aprotique polaire, tel que le diméthylformamide, à une température comprise entre 50°C et la température d'ébullition du solvant.

A partir des produits 3, les composés (I) s'obtiennent par les méthodes habituelles de désalkylation et notamment par action de l'acide bromhydrique en solution acétique ou par chauffage avec du chlorhydrate de pyridine.

Les composés méthylés 3 sont nouveaux et sont les intermédiaires clés de la préparation des composés (I). A ce titre, ils font partie intégrante de l'invention ainsi que leurs sels d'addition d'acides qui peuvent exister sous les deux formes tautomères:

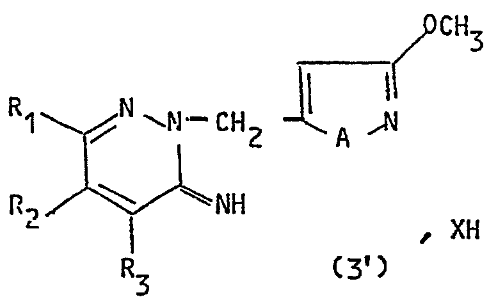

, XH (3')

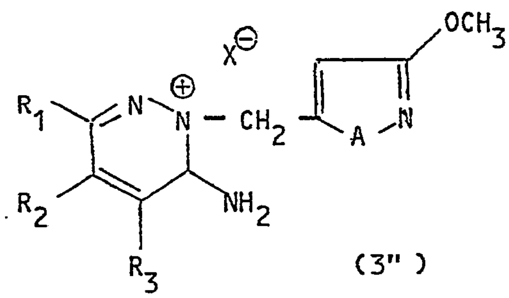

(3")

où $R_1$, $R_2$, $R_3$ et A sont tels que définis précédemment et X représente l'anion d'un acide minéral ou organique.

Les amino-3 pyridazines de départ 1 sont connues et peuvent être préparées à partir des chloro-3 pyridazines correspondantes, par exemple par action de l'hydrazine qui conduit aux dérivés hydrazino-3 correspondants. Ces derniers, par hydrogénation catalytique en présence de nickel de Raney, conduisent aux composés 1.

Les méthoxy-3 chloro (ou bromo) méthyl-5 isoxazole 2 s'obtiennent à partir de la méthoxy-3 hydroxyméthyl-5 isoxazole par action respectivement du chlorure de thionyle ou d'un dérivé bromé du phosphore tel que P $Br_3$.

Le méthoxy-3 hydroxyméthyl-5 isoxazole est un composé connu qui peut être préparé selon la méthode indiquée dans Acta Chemica Scandinavia Serie B 1976, B 30 (3), 281–2.

Le méthoxy-3 chlorométhyl-5 isothiazole est un composé connu qui peut être préparé selon la méthode décrite dans Journal of American Chemical Society 65, 1569 (1943).

Les exemples suivants permettront de mieux comprendre l'invention sans en limiter la portée. Dans ces exemples, les composés sont désignés par les références internes de la demanderesse.

EXEMPLE 1 :

((Hydroxy-3 isoxazolyl-5) méthyl)-2 imino-3 méthyl-4 phényl-6 dihydro-2,3 pyridazine, bromhydrate (SR 95538)

(I) $R_1$ = 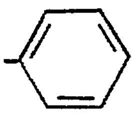 ; $R_2$ = H ; $R_3$ = $CH_3$ ; A = O

a) Chlorométhyl-5 méthoxy-3 isoxazole

On chauffe au reflux durant 30 min le mélange de 12,05 g d'hydroxyméthyl-5 méthoxy-3 isoxazole et 43 g de chlorure de thionyle. Après refroidissement, on verse lentement le mélange réactionnel sur de la glace pilée et on extrait avec de l'éther. On sépare la phase organique, lave avec de l'eau et sèche la solution sur sulfate de magnésium. On évapore le solvant puis distille sous vide. On obtient 9,64 g du produit attendu.
Eb / 0,2 mm de Hg = 30°C;
Rendement 70%.

b)Imino-3 [(méthoxy-3 isoxazolyl-5) méthyl]-2 méthyl-4 phényl-6 dihydro-2,3 pyridazine

A la solution de 1,85 g d'amino-3 méthyl-4 phényl-6 pyridazine dans 10 ml de diméthylformamide, on ajoute 1,62 g de chlorométhyl-5 méthoxy-3 isoxazole puis on chauffe à 80°C pendant 4 h.

Après refroidissement, on ajoute une solution saturée de bicarbonate de sodium et extrait avec de l'acétate d'éthyle. On sépare la phase organique, lave avec de l'eau salée puis sèche la solution sur sulfate de magnésium. On évapore le solvant sous vide. On chromatographie le produit brut sur colonne de silice et, en éluant avec de l'acétate d'éthyle, on obtient le produit attendu (1,77 g) sous forme d'une huile. Rendement 60%.

Par action de l'acide chlorhydrique gazeux, on transforme le produit en son chlorhydrate. F = 240°C avec décomposition.

c) SR 95538

On chauffe à 100°C pendant 4 h 1,5 g du produit obtenu ci-dessus sous forme de base dans 15 ml d'un mélange acide acétique-acide bromhydrique à 48% (50/50, vol/vol). Après concentration à petit volume, il se sépare 1,2 g du produit attendu. Le bromhydrate cristallise avec 0,5 molécule d'eau.
F = 164°C avec décomposition;
Rendement 65%.

EXEMPLE 2

[(Hydroxy-3 isothiazolyl-5) méthyl]-2 imino-3 (chloro-4 phényl)-6 dihydro-2,3 pyridazine, bromhydrate (SR 95885)

(I) $R_1$ = 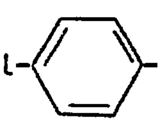 ; $R_2$ = $R_3$ = H ; A = S

a) [(Méthoxy-3 isothiazolyl-5) méthyl]-2 imino-3 (chloro-4 phényl)-6 dihydro-2,3 pyridazine, chlorhydrate

On chauffe sous argon à 80°C pendant 14 h le mélange de 0,500 g d'amino-3 (chloro-4 phényl)-6 pyridazine et 0,438 g de chlorométhylène-5 méthoxy-3 isothiazole dans 10 ml de diméthylformamide.

Après refroidissement, on essore le précipité et lave avec de l'acétone. On recristallise dans l'éthanol. On obtient des paillettes incolores.
F = 226°C;

Poids = 0,480 g;
Rendement 53%.

b) SR 95885

On libère la base du chlorhydrate obtenu ci-dessus par neutralisation avec de la soude normale et extraction au dichlorométhane. On sèche la solution et évapore le solvant.

On chauffe à 100°C pendant 3 h la solution de 0,400 g de la base dans 20 ml de mélange acide acétique-acide bromhydrique à 48% (50/50; vol/vol).

On évapore à siccité sous vide et reprend le résidu dans l'acétone. On obtient un solide incolore qu'on essore et recristallise dans l'éthanol.
F = 255°C;
Poids = 0,300 g;
Rendement 46%.

EXEMPLES 3 à 8

En opérant comme dans l'exemple 2, mais en faisant varier les réactifs, on prépare les composés I représentés dans le tableau suivant.

Dans chaque cas, on a indiqué la durée et la température de chauffage utilisées pour la déméthylation suivant l'exemple 2 b).

TABLEAU 1

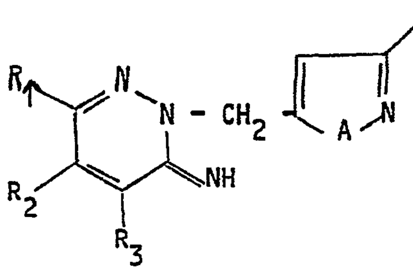
, HBr

| N° de code | $R_1$ | $R_2$ | $R_3$ | A | F = °C (1) | Déméthylation : température durée | N° Ex |
|---|---|---|---|---|---|---|---|
| 95884 | 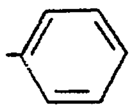 | H | H | 0 | 202 | 100°C - 12 h | 3 |
| 95812 | 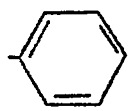 | H | H | 0 | 220 | 100°C - 6 h | 4 |
| 95895 | 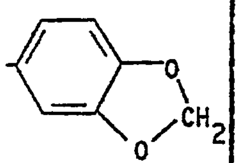 | H | H | 0 | 240 | 100°C - 3 h | 5 |
| 95886 | $-CH_3$ | 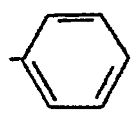 | H | S | 178 | 100°C - 4 h | 6 |
| 95893 | 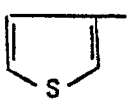 | H | H | 0 | 220 | 80 h - 2 h | 7 |
| 95894 | 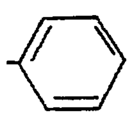 | -CH=CH-CH=CH | | 0 | 151 | 100°C-2h30min | 8 |

(1) Point de fusion après recristallisation dans l'éthanol.

L'activité thérapeutique des produits selon l'invention a été étudiée. Afin de la mettre en évidence, on a étudié l'activité neurochimique des produits sur le système GABA-ergique qui a été évaluée par la mesure du déplacement de l'acide gamma amino butyrique (GABA) de son récepteur post-synaptique.

L'étude a été effectuée selon la méthode de ENNA et SNYDER (Brain Research 100, 81-97, 1975). L'expérimentation a été effectuée in vitro en présence de suspension de membrane synaptique et de GA-BA tritié à la concentration finale de 2,9 nM.

Les résultats sont exprimés en Concentration Inhibitrice 50 (CI50), c'est-à-dire la concentration micromolaire qui inhibe 50% de la fixation du GABA sur son récepteur post-synaptique.

Les résultats obtenus avec divers produits de l'invention sont réunis dans le tableau 2.

Tableau 2

| Produit | | CI 50 en micromoles |
|---|---|---|
| No code SR | No exemple | |
| 95538 | 1 | 1 |
| 95885 | 2 | 0,15 |
| 95884 | 3 | 1,8 |
| 95812 | 4 | 1,2 |
| 95895 | 5 | 0,075 |
| 95886 | 6 | 6,6 |
| 95893 | 7 | 1,6 |
| 95894 | 8 | 0,12 |

Les produits selon l'invention agissent donc sur le neurone par occupation du site récepteur de l'acide gamma amino butyrique.

Leurs propriétés neurochimiques permettent donc d'envisager leur emploi en thérapeutique humaine et notamment comme antidépresseurs ou psychostimulants.

Les propriétés antidépressives des produits selon l'invention ont été étudiées chez l'animal. L'activité antidépressive a été évaluée dans le test d'antagonisme de la ptôse à la réserpine chez la souris.

L'étude a été effectuée sur des lots de 10 souris femelles pesant 20 ± 1 g. Les produits à étudier ont été administrés par voie intrapéritonéale en même temps que la réserpine par voie intraveineuse à la dose de 2 mg/kg.

Parallèlement, un lot témoin ne reçoit que le véhicule d'administration du produit à étudier et la réserpine.

1 h après l'administration, les animaux sont observés individuellement. Tous les animaux du lot témoin ont présenté la ptôse. Pour les lots traités, on compte les animaux ne présentant pas de ptôse et détermine le pourcentage des animaux où la ptôse a été antagonisée.

Ainsi, à la dose de 100 mg/kg par voie intrapéritonéale, le composé SR 95 538 inhibe la ptôse chez 30% des animaux.

Par ailleurs, les produits selon l'invention ne présentent pas de toxicité marquée aux doses où ils sont actifs. Ainsi, le produit SR 95 538 (exemple 1) ne révèle aucun signe de toxicité aiguë chez la souris à la dose de 100 mg/kg par voie intrapéritonéale. Les produits selon l'invention peuvent être administrés par voie orale ou par voie injectable.

Les compositions pharmaceutiques peuvent être solides ou liquides et se présenter par exemple sous forme de comprimés, gélules, granulés ou préparation injectable.

La posologie peut varier dans de larges proportions suivant le type et la gravité de l'affection à traiter et suivant le mode d'administration. Le plus souvent, chez l'adulte par voie orale, elle est comprise entre 0,010 g et 0,500 g par jour éventuellement répartie en plusieurs prises.

A titre d'exemple, on peut indiquer la préparation galénique suivante :

| Gélules | | |
|---|---|---|
| Produit de l'exemple 1 | 50 | mg |
| Aérosil | 0,5 | mg |
| Stéarate de magnésium | 1,5 | mg |
| Amidon STA RX 1500 | 48 | mg |
| | 100 | mg |

D'autre part, les produits selon l'invention étant des antagonistes puissants GABA A sélectifs peuvent être utilisés en tant que réactifs biologiques et notamment pour l'étude des systèmes biologiques où un mécanisme GABAergique est susceptible d'intervenir.

Enfin, on sait que pour différentes classes d'insecticides un mécanisme d'action en tant qu'inhibiteur du récepteur GABA A a été mis en évidence. On peut notamment se référer à l'article de L.J. LAWRENCE et J.E. CASSIDA, Life Science 35, 171, 1984 sur ce sujet. En tant qu'antagonistes puissants du récepteur GABA A, les produits selon l'invention possèdent une activité insecticide.

**Revendications pour les Etats contractants: BE, CH, DE, FR, GB, IT, LI, LU, NL, SE**

1. Dérivés de l'imino-3 dihydro-2,3 pyridazine, caractérisés en ce qu'ils répondent à la formule générale:

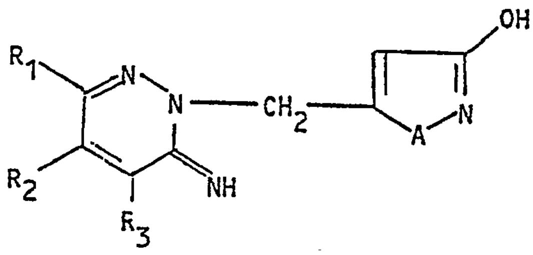

(I)

dans laquelle
– A représente un atome d'oxygène ou de soufre;
– $R_1$ représente un groupe alkyle en $C_1$–$C_4$, ou un groupe aromatique choisi parmi:
– le groupe phényle;
– les groupes phényle mono- ou poly-substitués par un groupe halogène, un groupe alkyle en $C_1$–$C_4$, un groupe alcoxy en $C_1$–$C_4$, un groupe nitro, un groupe hydroxy ou méthylènedioxy;
– le groupe naphtyle;
– le groupe furyle;
– le groupe thiényle ou le groupe pyridyle;
– $R_2$ et $R_3$ représentent chacun indépendamment l'hydrogène, un groupe alkyle en $C_1$–$C_4$, un groupe phényle ou encore $R_2$ et $R_3$ pris ensemble constituent, avec les 2 atomes du cycle pyridazinique auxquels ils sont liés, un noyau benzénique; et les sels d'addition de ces composés, avec des acides pharmaceutiquement acceptables, répondant aux formules I' ou I":

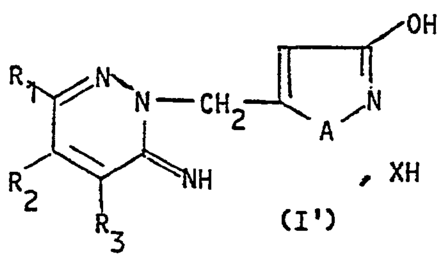

, XH

(I')

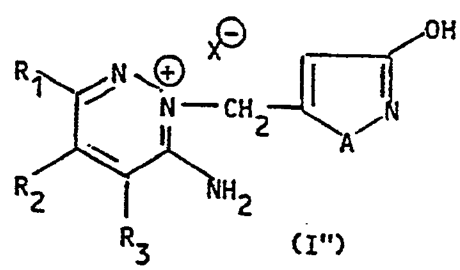

(I")

où $R_1$, $R_2$, $R_3$ et A sont tels que définis précédemment et X représente l'anion d'un acide minéral ou organique.

2. Dérivés selon la revendication 1, caractérisés en ce que $R_1$ est un groupe aromatique, $R_2$ et $R_3$ représentent l'hydrogène.

3. Dérivés selon la revendication 1, caractérisés en ce que $R_1$ est un alkyle en $C_1$–$C_4$, $R_2$ est le groupe phényle et $R_3$ est l'hydrogène.

4. Dérivés selon l'une quelconque des revendications 1 à 3, caractérisés en ce qu'ils se présentent sous la forme de sels d'acides halogénohydriques.

5. Dérivés de l'imino-3 dihydro-2,3 pyridazine, caractérisés en ce qu'ils répondent à la formule générale:

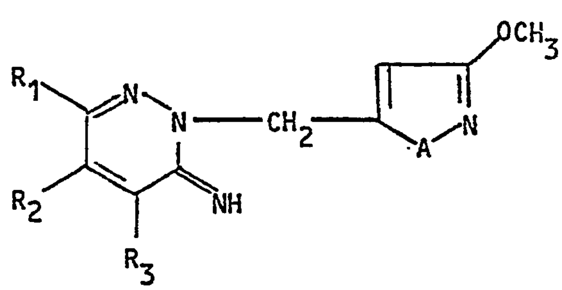

(3)

dans laquelle A, $R_1$, $R_2$ et $R_3$ sont tels que définis dans la revendication 1 et les sels d'addition d'acides desdits composés de formules:

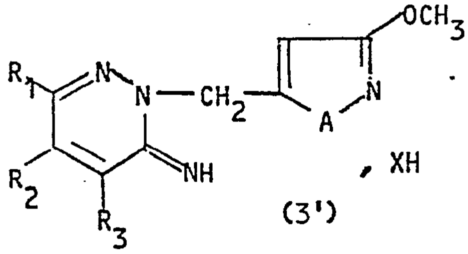

(3')

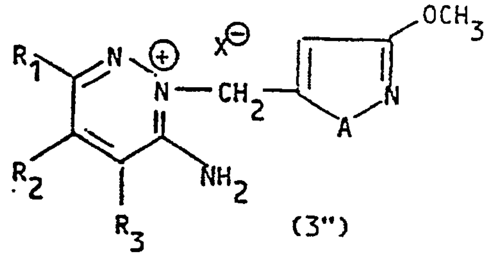

(3")

6. Procédé d'obtention des dérivés selon la revendication 1, caractérisé en ce que:
1) on fait réagir une amino-3 pyridazine de formule 1:

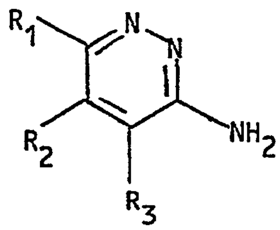

1

dans laquelle $R_1$, $R_2$ et $R_3$ sont tels que définis dans la revendication 1, avec un chlorométhyl-5 ou brométhyl-5 méthoxy-3 isoxazole ou isothiazole de formule 2:

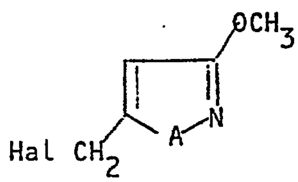

2

dans laquelle Hal représente le chlore ou le brome et A est tel que défini dans la revendication 1;
2) on déméthyle le composé méthoxylé ainsi obtenu;
3) et éventuellement on transforme les dérivés (I) ainsi obtenus en sels d'addition avec des acides.

7. Procédé selon la revendication 6, caractérisé en ce que la première réaction est effectuée dans un solvant aprotique polaire à une température comprise entre 50°C et la température d'ébullition du solvant.

8. Compositions pharmaceutiques actives, notamment sur le système nerveux central, caractérisées en ce qu'elles contiennent un dérivé de l'imino-3 dihydro-2,3 pyridazine selon l'une quelconque des revendications 1 à 4.

**Revendications pour les Etats contractants: AT, ES**

1. Procédé pour l'obtention de dérivés de l'imino-3 dihydro-2,3 pyridazine répondant à la formule:

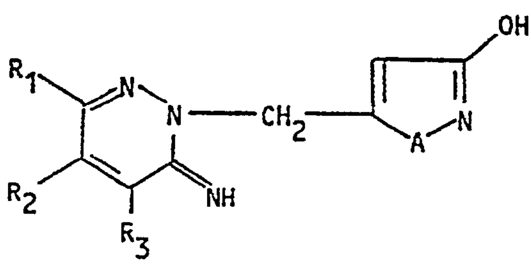

(I)

dans laquelle
– A représente un atome d'oxygène ou de soufre;
– $R_1$ représente un groupe alkyle en $C_1$–$C_4$, ou un groupe aromatique choisi parmi:
– le groupe phényle;
– les groupes phényle mono- ou poly-substitués par un groupe halogène, un groupe alkyle en $C_1$–$C_4$, un groupe alcoxy en $C_1$–$C_4$, un groupe nitro, un groupe hydroxy ou méthylènedioxy;
– le groupe naphtyle;
– le groupe furyle;

– le groupe thiényle ou le groupe pyridyle;

– $R_2$ et $R_3$ représentent chacun indépendamment l'hydrogène, un groupe alkyle en $C_1$–$C_4$, un groupe phényle ou encore $R_2$ et $R_3$ pris ensemble constituent, avec les 2 atomes du cycle pyridazinique auxquels ils sont liés, un noyau benzénique; et les sels d'addition de ces composés, avec des acides pharmaceutiquement acceptables, répondant aux formules I' ou I'':

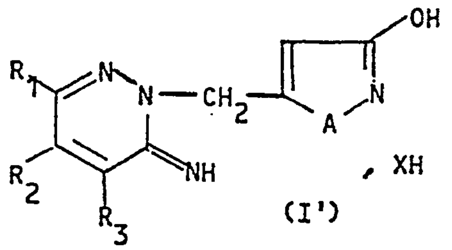

(I')

,

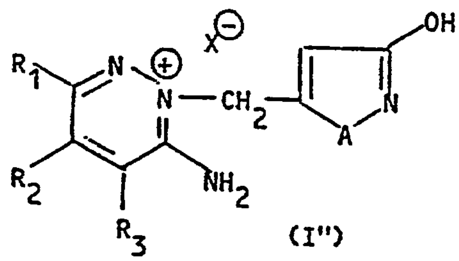

(I'')

où $R_1$, $R_2$, $R_3$ et A sont tels que définis précédemment et X représente l'anion d'un acide minéral ou organique, caractérisé en ce que:

1) on fait réagir une amino-3 pyridazine de formule 1:

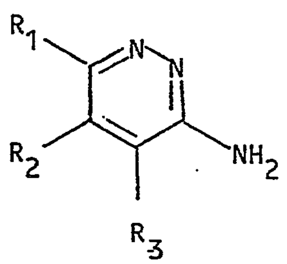

1

dans laquelle $R_1$, $R_2$ et $R_3$ sont tels que définis ci-dessus, avec un chlorométhyl-5 ou brométhyl-5 méthoxy-3 isoxazole ou isothiazole de formule 2:

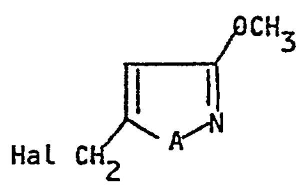

2

dans laquelle Hal représente le chlore ou le brome et A est tel que défini ci-dessus;

2) on déméthyle le composé méthoxylé ainsi obtenu;

3) et éventuellement on transforme les dérivés (I) ainsi obtenus en sels d'addition avec des acides.

2. Procédé selon la revendication 1, caractérisé en ce que l'on utilise un composé de formule 1 dans laquelle $R_1$ est un groupe aromatique, $R_2$ et $R_3$ représentent l'hydrogène.

3. Procédé selon la revendication 1, caractérisé en ce que l'on utilise un composé de formule 1 dans laquelle $R_1$ est un alkyle en $C_1$–$C_4$, $R_2$ est le groupe phényle et $R_3$ est l'hydrogène.

4. Procédé selon l'une quelconque des revendications 1 à 3, caractérisé en ce que lesdits dérivés (I) sont isolés sous forme de sels d'acides halogénohydriques.

5. Procédé selon l'une quelconque des revendications 1 à 4, caractérisé en ce que la première réaction est effectuée dans un solvant aprotique polaire à une température comprise entre 50°C et la température d'ébullition du solvant.

6. Procédé pour l'obtention de dérivés de l'imino-3 dihydro-2,3 pyridazine répondant à la formule

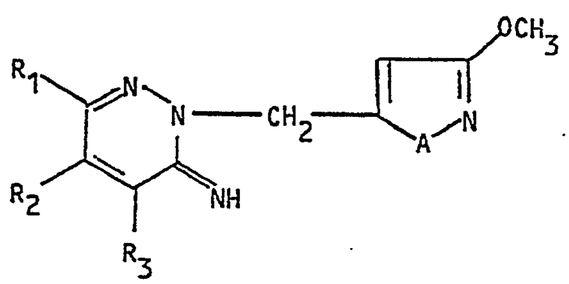

(3)

dans laquelle A, $R_1$, $R_2$ et $R_3$ sont tels que définis dans la revendication 1 et des sels d'addition d'acides desdits composés de formules:

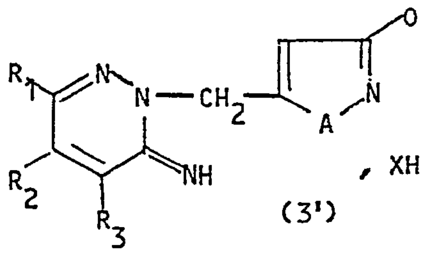

, XH

(3')

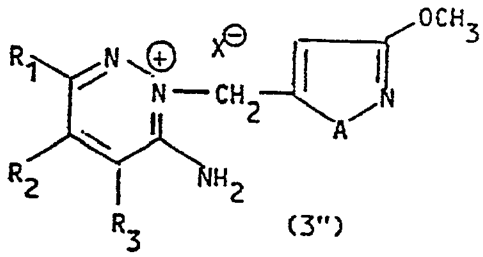

(3")

caractérisé en ce qu'il consiste à mettre en œuvre l'étape 1) du procédé selon l'une quelconque des revendications 1 à 5.

7. Procédé pour la préparation de compositions pharmaceutiques actives, notamment sur le système nerveux central, caractérisé en ce qu'il consiste à mélanger à des excipients convenables une dose efficace d'un dérivé de l'imino-3 dihydro-2,3 pyridazine préparé selon l'une quelconque des revendications 1 à 5.

**Revendications pour l'Etat contractant: GR**

1. Dérivés de l'imino-3 dihydro-2,3 pyridazine, caractérisés en ce qu'ils répondent à la formule générale:

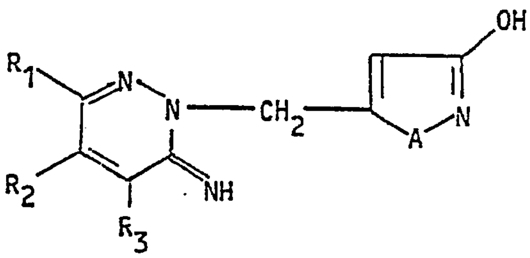

(I)

dans laquelle
- A représente un atome d'oxygène ou de soufre;
- $R_1$ représente un groupe alkyle en $C_1$–$C_4$, ou un groupe aromatique choisi parmi:
- le groupe phényle;
- les groupes phényle mono- ou poly-substitués par un groupe halogène, un groupe alkyle en $C_1$–$C_4$, un groupe alcoxy en $C_1$–$C_4$, un groupe nitro, un groupe hydroxy ou méthylènedioxy;
- le groupe naphtyle;
- le groupe furyle;
- le groupe thiényle ou le groupe pyridyle;
- $R_2$ et $R_3$ représentent chacun indépendamment l'hydrogène, un groupe alkyle en $C_1$–$C_4$, un groupe phényle ou encore $R_2$ et $R_3$ pris ensemble constituent, avec les 2 atomes du cycle pyridazinique auxquels ils sont liés, un noyau benzénique; et les sels d'addition de ces composés, avec des acides pharmaceutiquement acceptables, répondant aux formules I' ou I":

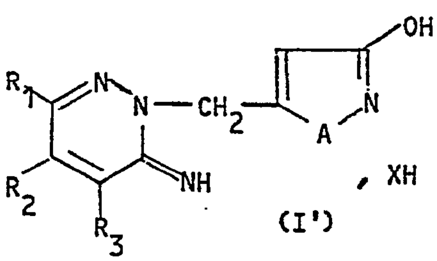

, XH

(I')

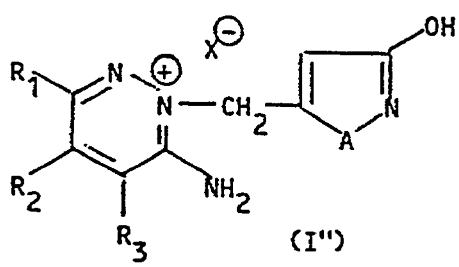

(I")

où $R_1$, $R_2$, $R_3$ et A sont tels que définis précédemment et X représente l'anion d'un acide minéral ou organique.

2. Dérivés selon la revendication 1, caractérisés en ce que $R_1$ est un groupe aromatique, $R_2$ et $R_3$ représentent l'hydrogène.

3. Dérivés selon la revendication 1, caractérisés en ce que $R_1$ est un alkyle en $C_1$–$C_4$, $R_2$ est le groupe phényle et $R_3$ est l'hydrogène.

4. Dérivés selon l'une quelconque des revendications 1 à 3, caractérisés en ce qu'ils se présentent sous la forme de sels d'acides halogénohydriques.

5. Dérivés de l'imino-3 dihydro-2,3 pyridazine, caractérisés en ce qu'ils répondent à la formule générale:

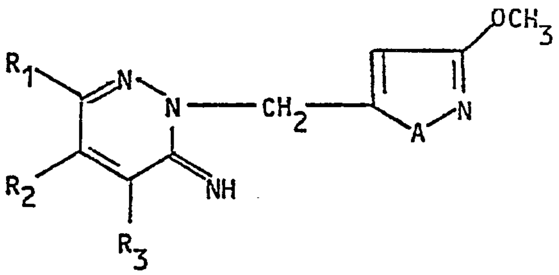

(3)

dans laquelle A, $R_1$, $R_2$ et $R_3$ sont tels que définis dans la revendication 1 et les sels d'addition d'acides desdits composés de formules:

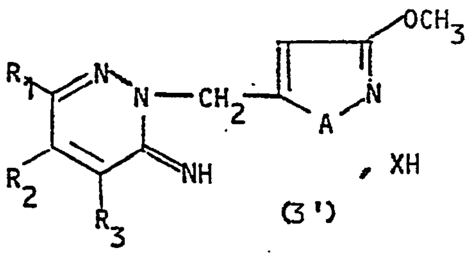

, XH

(3')

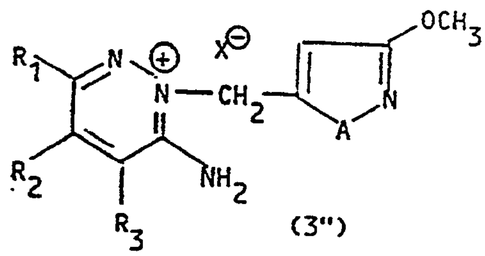

(3")

6. Procédé d'obtention des dérivés selon la revendication 1, caractérisé en ce que:

1) on fait réagir une amino-3 pyridazine de formule 1:

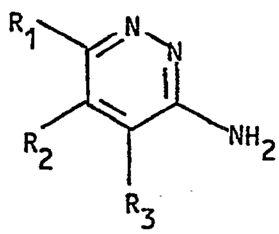

1

dans laquelle $R_1$, $R_2$ et $R_3$ sont tels que définis dans la revendication 1, avec un chlorométhyl-5 ou brométhyl-5 méthoxy-3 isoxazole ou isothiazole de formule 2:

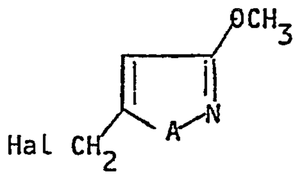

2

dans laquelle Hal représente le chlore ou le brome et A est tel que défini dans la revendication 1;

2) on déméthyle le composé méthoxylé ainsi obtenu;

3) et éventuellement on transforme les dérivés (I) ainsi obtenus en sels d'addition avec des acides.

7. Procédé selon la revendication 6, caractérisé en ce que la première réaction est effectuée dans un solvant aprotique polaire à une température comprise entre 50°C et la température d'ébullition du solvant.

8. Procédé pour la préparation compositions pharmaceutiques actives, notamment sur le système nerveux central, caractérisé en ce qu'il consiste à mélanger à des excipients convenables, une dose efficace d'un dérivé de l'imino-3 dihydro-2,3 pyridazine selon l'une quelconque des revendications 1 à 4.

**Patentansprüche für die benannten Vertragsstaaten: BE, CH, DE, FR, GB, IT, LI, LU, NL, SE:**

1. 3-Imino-2,3-dihydro-pyridazinderivate, dadurch gekennzeichnet, daß sie der allgemeinen Formel

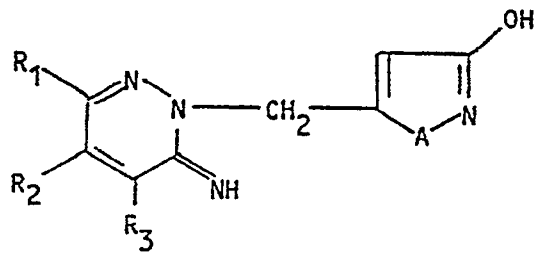

(I)

entsprechen, worin
– A ein Sauerstoff- oder Schwefelatom darstellt,
– $R_1$ eine $C_1$–$C_4$-Alkylgruppe oder eine aromatische Gruppe, ausgewählt aus:
– der Phenylgruppe,
– den durch ein Halogen, eine $C_1$–$C_4$-Alkylgruppe, eine $C_1$–$C_4$-Alkoxygruppe, eine Nitrogruppe, eine Hydroxy- oder Methylendioxygruppe mono- oder polysubstituierten Phenylgruppen;
– der Naphthylgruppe;
– der Furylgruppe;
– der Thienylgruppe oder der Pyridylgruppe darstellt;
– $R_2$ und $R_3$ jeweils unabhängig Wasserstoff, eine $C_1$–$C_4$-Alkylgruppe, eine Phenylgruppe darstellen, oder aber $R_2$ und $R_3$, zusammen genommen, mit den 2 Atomen des Pyridazinringes, an den sie gebunden sind, einen Benzolring bilden; und die Additionssalze dieser Verbindungen mit pharmazeutisch akzeptablen Salzen, entsprechend den Formeln I′ oder I″:

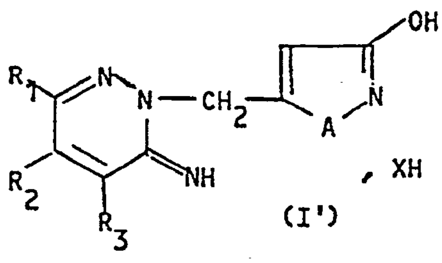

(I')

,

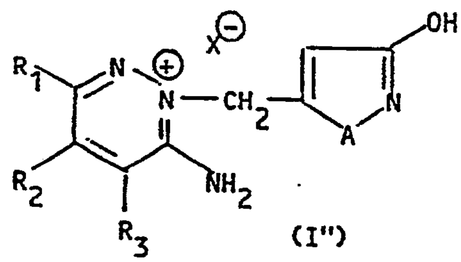

(I")

worin $R_1$, $R_2$, $R_3$ und A wie zuvor definiert sind und X das Anion einer Mineral- oder organischen Säure bezeichnet.

2. Derivate nach Anspruch 1, dadurch gekennzeichnet, daß $R_1$ eine aromatische Gruppe ist, $R_2$ und $R_3$ Wasserstoff darstellen.

3. Derivate nach Anspruch 1, dadurch gekennzeichnet, daß $R_1$ $C_1$–$C_4$-Alkyl ist, $R_2$ die Phenylgruppe ist und $R_3$ Wasserstoff ist.

4. Derivate nach einem der Ansprüche 1 bis 3, dadurch gekennzeichnet, daß sie in Form der Halogenwasserstoffsäuren vorliegen.

5. 3-Imino-2,3-dihydro-pyridazinderivate, dadurch gekennzeichnet, daß sie der allgemeinen Formel

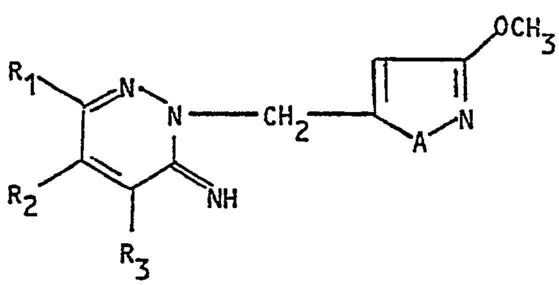

(3)

entsprechen, worin A, $R_1$, $R_2$ und $R_3$ wie in Anspruch 1 definiert sind, und die Säureadditionssalze der Verbindungen der Formeln

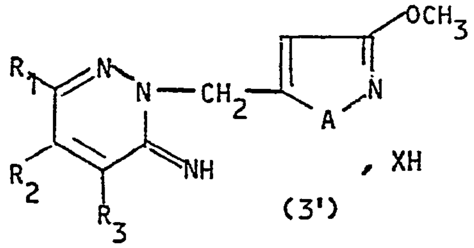

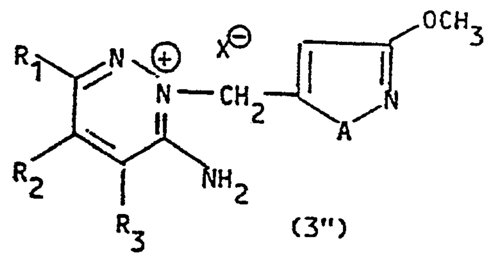

6. Verfahren zur Herstellung der Derivate nach Anspruch 1, dadurch gekennzeichnet, daß
1) man ein 3-Aminopyridazin der Formel 1:

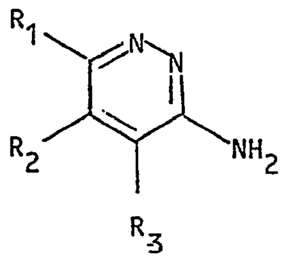

1

worin $R_1$, $R_2$ und $R_3$ wie in Anspruch 1 definiert sind, mit einem 5-Chlormethyl- oder 5-Brommethyl-3-methoxy-isoxazol oder -isothiazol der Formel 2:

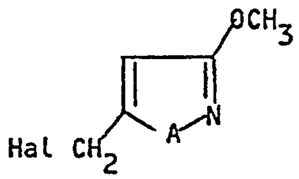

2

worin Hal für Chlor oder Brom steht und A wie in Anspruch 1 definiert ist, zur Umsetzung bringt;
2) man die so erhaltene Methoxylverbindung entmethyliert;
3) und man gegebenenfalls die so erhaltenen Derivate (I) mit Säuren in Additionssalze überführt.

7. Verfahren nach Anspruch 6, dadurch gekennzeichnet, daß die erste Reaktion in einem aprotischen polaren Lösungsmittel bei einer Temperatur zwischen 50°C und der Kochtemperatur des Lösungsmittels durchgeführt wird.

8. Pharmazeutische Zusammensetzungen, die insbesondere auf das Zentralnervensystem aktiv sind, dadurch gekennzeichnet, daß sie ein 3-Imino-2,3-dihydro-pyridazinderivat nach einem der Ansprüche 1 bis 4 enthalten.

**Patentansprüche für die benannten Vertragstaaten: AT, ES**

1. Verfahren zur Herstellung von 3-Imino-2,3-dihydro-pyridazinderivaten der Formel

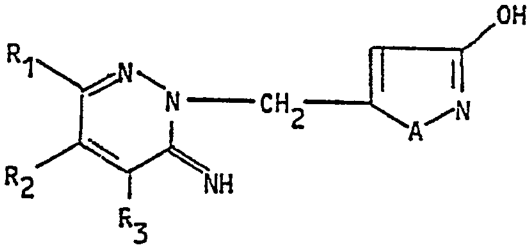

(I)

worin
– A ein Sauerstoff- oder Schwefelatom darstellt,
– $R_1$ eine $C_1$–$C_4$-Alkylgruppe oder eine aromatische Gruppe, ausgewählt aus:
– der Phenylgruppe,
– den durch ein Halogen, eine $C_1$–$C_4$-Alkylgruppe, eine $C_1$–$C_4$-Alkoxygruppe, eine Nitrogruppe, eine Hydroxy- oder Methylendioxygruppe mono- oder polysubstituierten Phenylgruppen;
– der Naphthylgruppe;

– der Furylgruppe;
– der Thienylgruppe oder der Pyridylgruppe darstellt;
– $R_2$ und $R_3$ jeweils unabhängig Wasserstoff, eine $C_1$–$C_4$-Alkylgruppe, eine Phenylgruppe darstellen, oder aber $R_2$ und $R_3$, zusammen genommen, mit den 2 Atomen des Pyridazinringes, an den sie gebunden sind, einen Benzolring bilden; und der Additionsalze dieser Verbindungen mit pharmazeutisch akzeptablen Salzen, entsprechend den Formeln I' oder I'':

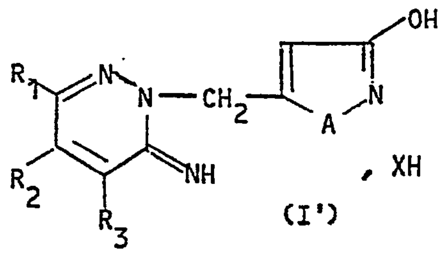
, XH (I')

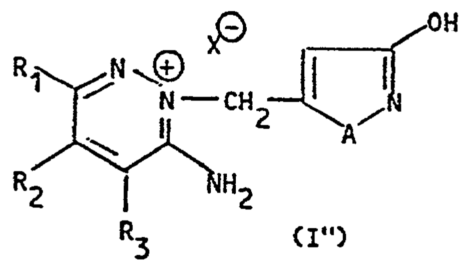
(I'')

worin $R_1$, $R_2$, $R_3$ und A wie zuvor definiert sind und X das Anion einer Mineral- oder organischen Säure bezeichnet, dadurch gekennzeichnet, daß

1) man ein 3-Aminopyridazin der Formel 1:

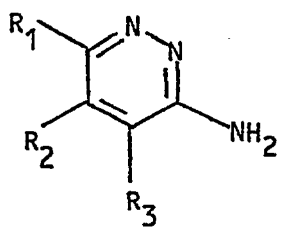
1

worin $R_1$, $R_2$ und $R_3$ wie oben definiert sind, mit einem 5-Chlormethyl- oder 5-Brommethyl-3-methoxy-isoxazol oder -isothiazol der Formel 2:

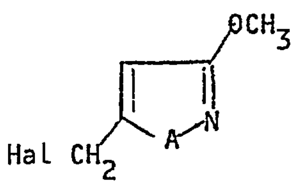
2

worin Hal für Chlor oder Brom steht und A wie oben definiert ist, zur Umsetzung bringt;
2) man die so erhaltene Methoxylverbindung entmethyliert;
3) und man gegebenenfalls die so erhaltenen Derivate (I) mit Säuren in Additionssalze überführt.

2. Verfahren nach Anspruch 1, dadurch gekennzeichnet, daß man eine Verbindung der Formel 1, worin $R_1$ eine aromatische Gruppe ist, $R_2$ und $R_3$ Wasserstoff darstellen, verwendet.

3. Verfahren nach Anspruch 1, dadurch gekennzeichnet, daß man eine Verbindung der Formel 1, worin $R_1$ $C_1$–$C_4$-Alkyl ist, $R_2$ die Phenylgruppe ist und $R_3$ Wasserstoff ist, verwendet.

4. Verfahren nach einem der Ansprüche 1 bis 3, dadurch gekennzeichnet, daß die Derivate (I) in Form von Halogenwasserstoffsäuren isoliert werden.

5. Verfahren nach einem der Ansprüche 1 bis 4, dadurch gekennzeichnet, daß die erste Reaktion in einem aprotischen polaren Lösungsmittel bei einer Temperatur zwischen 50°C und der Kochtemperatur des Lösungsmittels durchgeführt wird.

6. Verfahren zur Herstellung von 3-Imino-2,3-dihydro-pyridazinderivaten der Formel

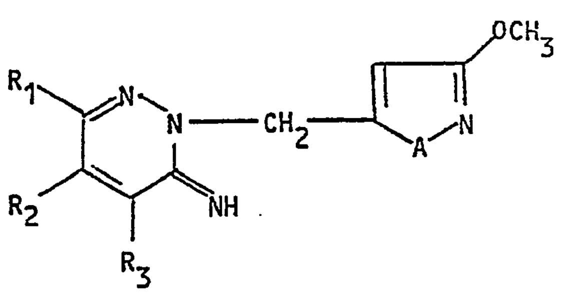
(3)

worin A, $R_1$, $R_2$ und $R_3$ wie in Anspruch 1 definiert sind, und der Säureadditionssalze der Verbindungen der Formeln

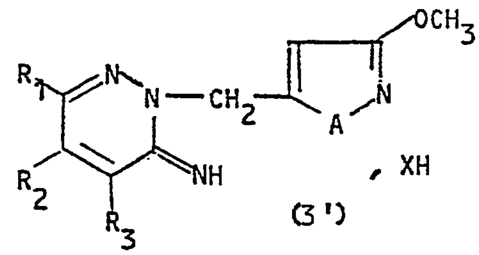

, XH (3')

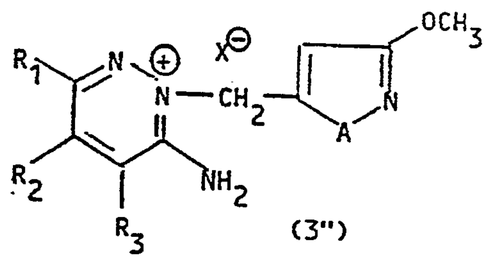

(3")

dadurch gekennzeichnet, daß es darin besteht, daß die Stufe 1) des Verfahrens nach einem der Ansprüche 1 bis 5 durchgeführt wird.

7. Verfahren zur Herstellung von pharmazeutischen Zusammensetzungen, die insbesondere auf das Zentralnervensystem aktiv sind, dadurch gekennzeichnet, daß es darin besteht, daß mit geeigneten Exzipienten eine wirksame Dosis eines 3-Imino-2,3-dihydro-pyridazinderivats, hergestellt nach einem der Ansprüche 1 bis 5, vermischt wird.

**Patentansprüche für den benannten Vertragsstaat: GR**

1. 3-Imino-2,3-dihydro-pyridazinderivate, dadurch gekennzeichnet, daß sie der allgemeinen Formel

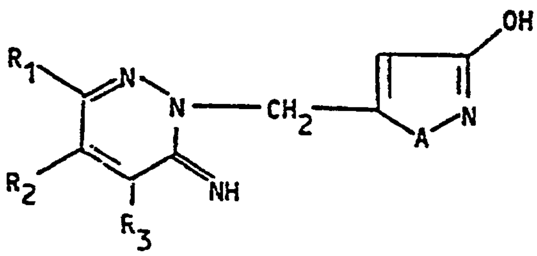

(I)

entsprechen, worin

- A ein Sauerstoff- oder Schwefelatom darstellt,
- $R_1$ eine $C_1$–$C_4$-Alkylgruppe oder eine aromatische Gruppe, ausgewählt aus:
- der Phenylgruppe,
- den durch ein Halogen, eine $C_1$–$C_4$-Alkylgruppe, eine $C_1$–$C_4$-Alkoxygruppe, eine Nitrogruppe, eine Hydroxy- oder Methylendioxygruppe mono- oder polysubstituierten Phenylgruppen;
- der Naphthylgruppe;
- der Furylgruppe;
- der Thienylgruppe oder der Pyridylgruppe darstellt;
- $R_2$ und $R_3$ jeweils unabhängig Wasserstoff, eine $C_1$–$C_4$-Alkylgruppe, eine Phenylgruppe darstellen, oder aber $R_2$ und $R_3$, zusammen genommen, mit den 2 Atomen des Pyridazinringes, an den sie gebunden sind, einen Benzolring bilden; und die Additionsalze dieser Verbindungen mit pharmazeutisch akzeptablen Salzen, entsprechend den Formeln I′ oder I″:

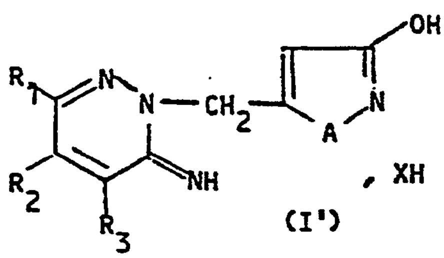

, XH (I')

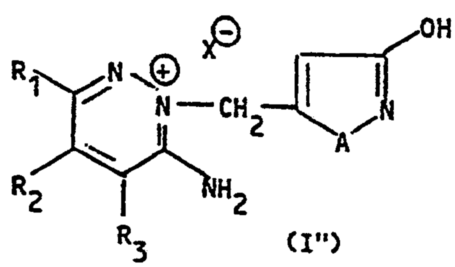

(I")

worin $R_1$, $R_2$, $R_3$ und A wie zuvor definiert sind und X das Anion einer Mineral- oder organischen Säure bezeichnet.

2. Derivate nach Anspruch 1, dadurch gekennzeichnet, daß $R_1$ eine aromatische Gruppe ist, $R_2$ und $R_3$ Wasserstoff darstellen.

3. Derivate nach Anspruch 1, dadurch gekennzeichnet, daß $R_1$ $C_1$–$C_4$-Alkyl ist, $R_2$ die Phenylgruppe ist und $R_3$ Wasserstoff ist.

4. Derivate nach einem der Ansprüche 1 bis 3, dadurch gekennzeichnet, daß sie in Form der Halogenwasserstoffsäuren vorliegen.

5. 3-Imino-2,3-dihydro-pyridazinderivate, dadurch gekennzeichnet, daß sie der allgemeinen Formel

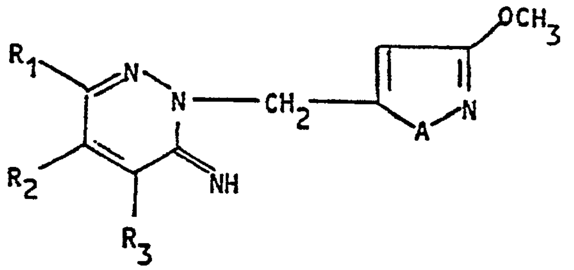

(3)

entsprechen, worin A, $R_1$, $R_2$ und $R_3$ wie in Anspruch 1 definiert sind, und die Säureadditionssalze der Verbindungen der Formeln

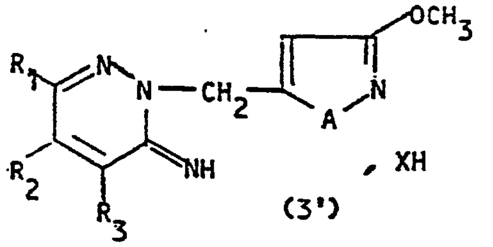

, XH

(3')

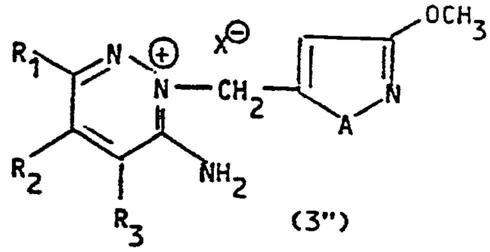

(3")

6. Verfahren zur Herstellung der Derivate nach Anspruch 1, dadurch gekennzeichnet, daß

1) man ein 3-Aminopyridazin der Formel 1:

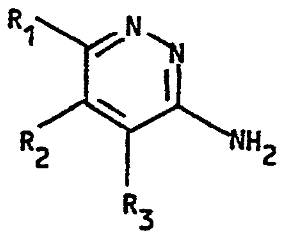

1

worin $R_1$, $R_2$ und $R_3$ wie in Anspruch 1 definiert sind, mit einem 5-Chlormethyl- oder 5-Brommethyl-3-methoxy-isoxazol oder -isothiazol der Formel 2:

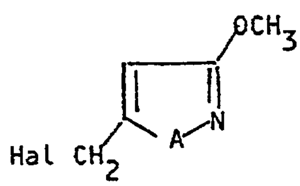

2

worin Hal für Chlor oder Brom steht und A wie in Anspruch 1 definiert ist, zur Umsetzung bringt;

2) man die so erhaltene Methoxylverbindung entmethyliert;

3) und man gegebenenfalls die so erhaltenen Derivate (I) mit Säuren in Additionssalze überführt.

7. Verfahren nach Anspruch 6, dadurch gekennzeichnet, daß die erste Reaktion in einem aprotischen polaren Lösungsmittel bei einer Temperatur zwischen 50°C und der Kochtemperatur des Lösungsmittels durchgeführt wird.

8. Verfahren zur Herstellung von pharmazeutischen Zusammensetzungen, die insbesondere auf das Zentralnervensystem aktiv sind, dadurch gekennzeichnet, daß es darin besteht, daß mit geeigneten Exzipienten eine wirksame Dosis eines 3-Imino-2,3-dihydro-pyridazinderivats nach einem der Ansprüche 1 bis 4 vermischt wird.

**Claims for the Contracting States: BE, CH, DE, FR, GB, IT, LI, LU, NL, SE**

1. Derivatives of 3-imino-2,3-dihydro-pyridazine, characterized in that they respond to the general formula:

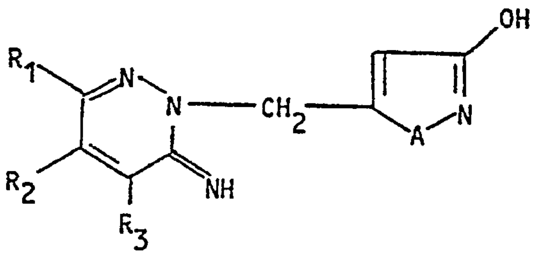

(I)

in which:
– A represents an atom of oxygen or of sulfur;
– $R_1$ represents a $C_1$–$C_4$ alkyl group, or an aromatic group selected from:
. the phenyl group;
. the phenyl groups mono- or poly-substituted by a halogen group, a $C_1$–$C_4$ alkyl group, a $C_1$–$C_4$ alkoxy group, a nitro group, a hydroxy or methylenedioxy group;
. the naphthyl group;
. the furyl group;
. the thienyl group or the pyridyl group;
– $R_2$ and $R_3$ each represent independently hydrogen, a $C_1$–$C_4$ alkyl group; a phenyl group, or $R_2$ and $R_3$, taken together, constitute, with the 2 atoms of the pyridazinic cycle to which they are bonded, a benzene ring; and the addition salts of these compounds, with pharmaceutically acceptable acids, responding to formulae I′ or I″;

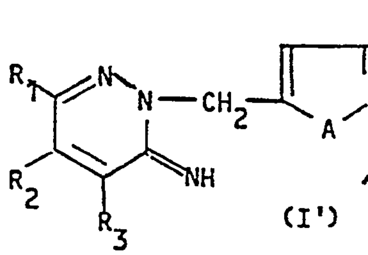

, XH

(I′)

,

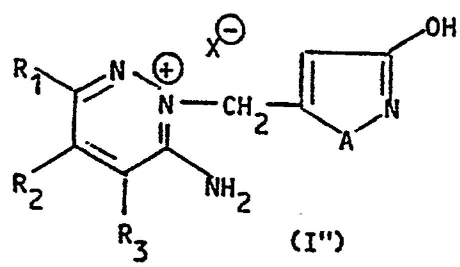

(I″)

in which $R_1$, $R_2$, $R_3$ and A are as defined hereinabove and X represents the anion of an inorganic or organic acid.

2. The derivatives according to claim 1, characterized in that $R_1$ is an aromatic group, $R_2$ and $R_3$ represent hydrogen.

3. The derivatives according to claim 1, characterized in that $R_1$ is a $C_1$–$C_4$ alkyl, $R_2$ is the phenyl group and $R_3$ is hydrogen.

4. The derivatives according to any one of claims 1 to 3, characterized in that they are in the form of halohydric acid salts.

5. Derivatives of 3-imino-2,3-dihydro-pyridazine, characterized in that they respond to the general formula:

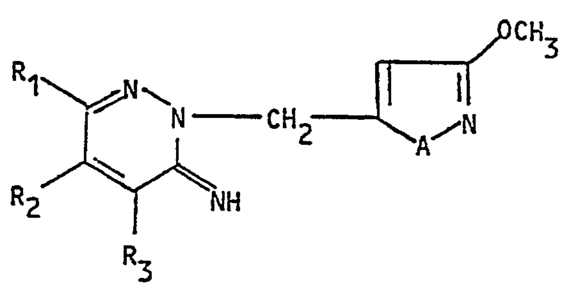

(3)

in which A, $R_1$, $R_2$ and $R_3$ are as defined in claim 1 and the acid addition salts of said compounds of formulae:

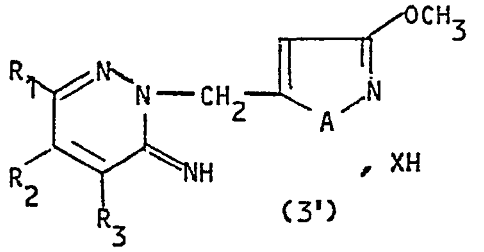

, XH (3')

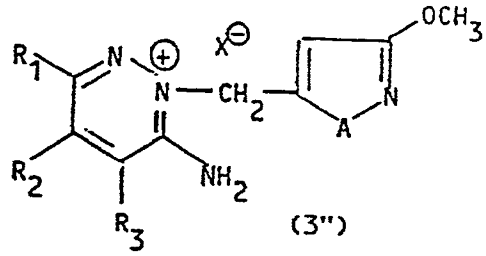

(3'')

6. Process for obtaining the derivatives according to claims 1, characterized in that it consists in:
1) reacting a 3-amino-pyridazine of formula 1:

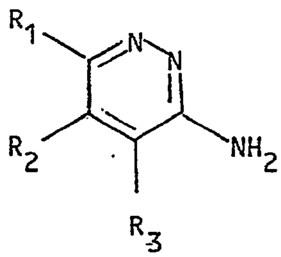

1

in which $R_1$, $R_2$ and $R_3$ are as defined in claim 1, with a 5-chloromethyl- or 5-bromethyl-3-methoxy-isoxazole or isothiazole of formula 2:

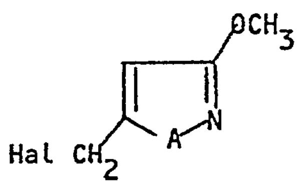

2

in which Hal represents chlorine or bromine and A is as defined in claim 1;
2) demethylating the methoxylated compound thus obtained;
3) and possibly converting the derivatives (I) thus obtained into addition salts with acids.

7. The process according to claim 6, characterized in that the first reaction is effected in a polar aprotic solvent at a temperature of between 50°C and the temperature of boiling of the solvent.

8. Pharmaceutical compositions active in particular on the central nervous system, characterized in that they contain a derivative of 3-imino-2,3-dihydro-pyridazine according to any one of claims 1 to 4.

**Claims for the Contracting States: AT, ES**

1. Process for obtaining derivatives of 3-imino-2,3-dihydro-pyridazine, responding to formula:

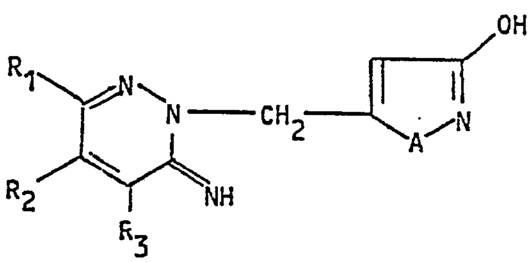

(I)

in which:
– A represents an atom of oxygen or of sulfur;
– $R_1$ represents a $C_1$–$C_4$ alkyl group, or an aromatic group selected from:
. the phenyl group;
. the phenyl groups mono- or poly-substituted by a halogen group, a $C_1$–$C_4$ alkyl group, a $C_1$–$C_4$ alkoxy group, a nitro group, a hydroxy or methylenedioxy group;
. the naphthyl group;
. the furyl group;
. the thienyl group or the pyridyl group;

– $R_2$ and $R_3$ each represent independently hydrogen, a $C_1$–$C_4$ alkyl group; a phenyl group, or $R_2$ and $R_3$, taken together, constitute, with the 2 atoms of the pyridazinic cycle to which they are bonded, a benzene ring; and the addition salts of these compounds, with pharmaceutically acceptable acids, responding to formulae I' or I'':

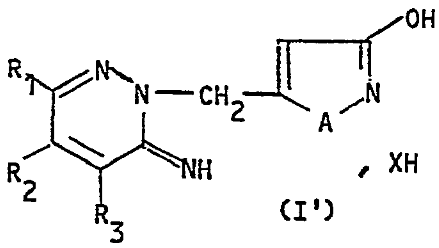

, XH

(I')

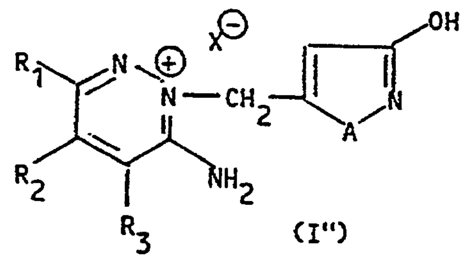

(I'')

in which $R_1$, $R_2$, $R_3$ and A are as defined hereinabove and X represents the anion of an inorganic or organic acid, characterized in that it comprises the steps of:

1) reacting a 3-amino-pyridazine of formula 1:

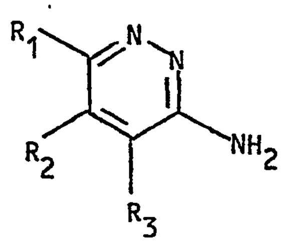

1

in which $R_1$, $R_2$ and $R_3$ are as defined hereinabove, with a 5-chloromethyl- or 5-bromethyl-3-methoxy-isoxazole or isothiazole of formula 2:

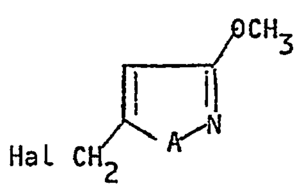

2

in which Hal represents chlorine or bromine and A is as defined hereinabove;

2) demethylating the methoxylated compound thus obtained;

3) and possibly converting the derivatives (I) thus obtained into addition salts with acids.

2. The process according to claim 1, characterized in that a compound of formula 1 is used in which $R_1$ is an aromatic group, $R_2$ and $R_3$ represent hydrogen.

3. The process according to claim 1, characterized in that a compound of formula 1 is used in which $R_1$ is a $C_1$–$C_4$ alkyl, $R_2$ is the phenyl group and $R_3$ is hydrogen.

4. The process according to any one of claims 1 to 3, characterized in that said derivatives (I) are isolated in the form of hydrohalic acid salts.

5. The process according to any one of claims 1 to 4, characterized in that the first reaction is effected in a polar aprotic solvent at a temperature of between 50°C and the temperature of boiling of the solvent.

6. The process for obtaining derivatives of 3-imino-2,3-dihydro-pyridazine, responding to formula:

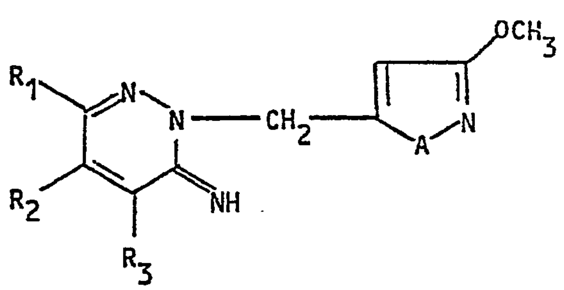

(3)

in which A, $R_1$, $R_2$ and $R_3$ are as defined in claim 1, and the acid addition salts of said compounds of formulae:

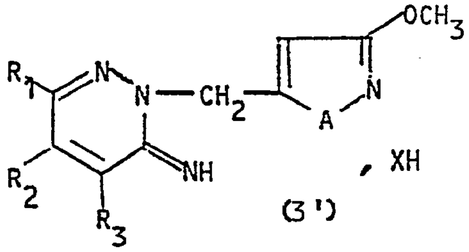

, XH

(3')

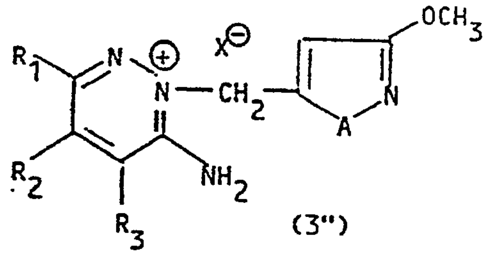

(3")

characterized in that they consist in carrying out step 1) of the process according to any one of claims 1 to 5.

7. Process for the preparation of pharmaceutical compositions active in particular on the central nervous system, characterized in that it consists in mixing to appropriate excipients an active dose of a derivative of 3-imino-2,3-dihydro-pyridazine prepared according to any one of claims 1 to 5.

**Claims for the Contracting State: GR**

1. Derivatives of 3-imino-2,3-dihydro-pyridazine, characterized in that they respond to the general formula:

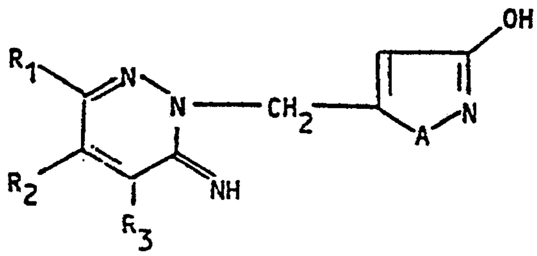

(I)

in which:

– A represents an atom of oxygen or of sulfur;

– $R_1$ represents a $C_1$–$C_4$ alkyl group, or an aromatic group selected from:

. the phenyl group;

. the phenyl groups mono- or poly-substituted by a halogen group, a $C_1$–$C_4$ alkyl group, a $C_1$–$C_4$ alkoxy group, a nitro group, a hydroxy or methylenedioxy group;

. the naphthyl group;

. the furyl group;

. the thienyl group or the pyridyl group;

– $R_2$ and $R_3$ each represent independently hydrogen, a $C_1$–$C_4$ alkyl group; a phenyl group, or $R_2$ and $R_3$, taken together, constitute, with the 2 atoms of the pyridazinic cycle to which they are bonded, a benzene ring; and the addition salts of these compounds, with pharmaceutically acceptable acids, responding to formulae I′ or I″:

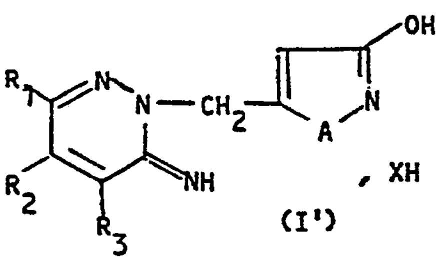

, XH

(I')

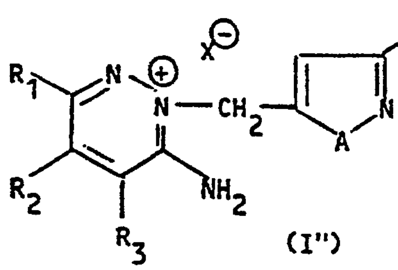

(I")

in which $R_1$, $R_2$, $R_3$ and A are as defined herein above and X represents the anion of an inorganic or organic acid.

2. The derivatives according to claim 1, characterized in that $R_1$ is an aromatic group, $R_2$ and $R_3$ represent hydrogen.

3. The derivatives according to claim 1, characterized in that $R_1$ is a $C_1$–$C_4$ alkyl, $R_2$ is the phenyl group and $R_3$ is hydrogen.

4. The derivatives according to any one of claims 1 to 3, characterized in that they are in the form of halohydric acid salts.

5. Derivatives of 3-imino-2,3-dihydro-pyridazine, characterized in that they respond to the general formula:

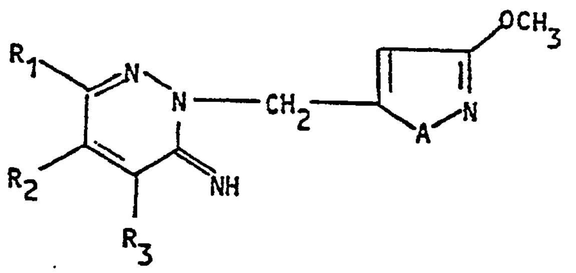

(3)

in which A, $R_1$, $R_2$ and $R_3$ are as defined in claim 1 and the acid addition salts of said compounds of formulae:

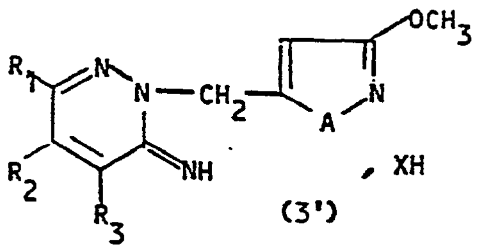

, XH (3')

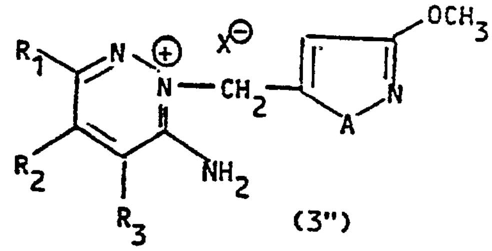

(3")

6. Process for obtaining the derivatives according to claims 1, characterized in that it consists in:
1) reacting a 3-amino-pyridazine of formula 1:

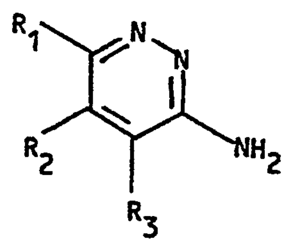

1

in which $R_1$, $R_2$ and $R_3$ are as defined in claim 1, with a 5-chloromethyl- or 5-bromethyl-3-methoxy-isoxazole or isothiazole of formula 2:

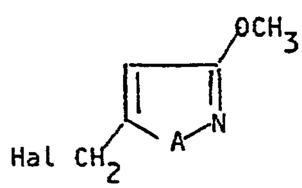

2

in which Hal represents chlorine or bromine and A is as defined in claim 1;
2) demethylating the methoxylated compound thus obtained;
3) and possibly converting the derivatives (I) thus obtained into addition salts with acids.

7. The process according to claim 6, characterized in that the first reaction is effected in a polar aprotic solvent at a temperature of between 50°C and the temperature of boiling of the solvent.

8. Process for the preparation of pharmaceutical compositions active in particular on the central nervous system, characterized in that it consists in mixing to appropriate excipients an active dose of a derivative of 3-imino-2,3-dihydro-pyridazine according to any one of claims 1 to 4.